(19) Europäisches Patentamt
European Patent Office
Office européen des brevets

(11) EP 3 333 262 A1

# (12) EUROPEAN PATENT APPLICATION
published in accordance with Art. 153(4) EPC

(43) Date of publication:
**13.06.2018 Bulletin 2018/24**

(21) Application number: **16833038.9**

(22) Date of filing: **02.08.2016**

(51) Int Cl.:
***C12N 15/09*** (2006.01) ***C07K 1/22*** (2006.01)
***C07K 14/31*** (2006.01) ***C12N 1/15*** (2006.01)
***C12N 1/19*** (2006.01) ***C12N 1/21*** (2006.01)
***C12N 5/10*** (2006.01) ***C12P 21/02*** (2006.01)

(86) International application number:
**PCT/JP2016/072649**

(87) International publication number:
**WO 2017/022759 (09.02.2017 Gazette 2017/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **04.08.2015 JP 2015154365**

(71) Applicant: **Kaneka Corporation**
**Osaka-shi, Osaka 530-8288 (JP)**

(72) Inventors:
- **NISHIHACHIJYO, Masakatsu**
**Takasago-shi**
**Hyogo 676-8688 (JP)**
- **YAMASHITA, Keita**
**Settsu-shi**
**Osaka 566-0072 (JP)**
- **ODAWARA, Osamu**
**Takasago-shi**
**Hyogo 676-8688 (JP)**
- **TSUCHIDATE, Takeyuki**
**Takasago-shi**
**Hyogo 676-8688 (JP)**
- **TAKANO, Masayuki**
**Takasago-shi**
**Hyogo 676-8688 (JP)**
- **YOSHIDA, Shinichi**
**Takasago-shi**
**Hyogo 676-8688 (JP)**

(74) Representative: **Hoffmann Eitle**
**Patent- und Rechtsanwälte PartmbB**
**Arabellastraße 30**
**81925 München (DE)**

## (54) IMMUNOGLOBULIN-BINDING MODIFIED PROTEIN

(57) The present invention provides an antibody-binding protein which can be produced by a transformant with improved culture productivity. The present invention relates to a protein having two or more amino acid sequences derived from any of the E, D, A, B, and C domains of Protein A of SEQ ID NOs:1 to 5, wherein in the domain-derived amino acid sequence closest to the N-terminus, one or more amino acid residues corresponding to position 4 and/or position 7 of the C domain are substituted by amino acid residues other than Arg, and wherein the protein can be produced by the transformant with improved culture productivity as compared to before the substitution.

EP 3 333 262 A1

SEQUENCE LISTING

<110> KANEKA CORPORATION

<120> IMMUNOGLOBULIN-BINDING MODIFIED PROTEIN

<130> B150467WO01

<140> PCT/JP2016/072649

<141> 2016-08-02

<150> JP 2015-154365

<151> 2015-08-04

<160> 22

<170> PatentIn version 3.5

<210> 1
<211> 56
<212> PRT
<213> Staphylococcus aureus

<400> 1

```
Ala Gln His Asp Glu Ala Gln Gln Asn Ala Phe Tyr Gln Val Leu Asn
1               5                   10                  15

Met Pro Asn Leu Asn Ala Asp Gln Arg Asn Gly Phe Ile Gln Ser Leu
            20                  25                  30

Lys Asp Asp Pro Ser Gln Ser Ala Asn Val Leu Gly Glu Ala Gln Lys
        35                  40                  45

Leu Asn Asp Ser Gln Ala Pro Lys
    50                  55
```

<210> 2
<211> 61
<212> PRT
<213> Staphylococcus aureus

<400> 2

```
Ala Asp Ala Gln Gln Asn Lys Phe Asn Lys Asp Gln Gln Ser Ala Phe
1               5                   10                  15

Tyr Glu Ile Leu Asn Met Pro Asn Leu Asn Glu Glu Gln Arg Asn Gly
            20                  25                  30

Phe Ile Gln Ser Leu Lys Asp Asp Pro Ser Gln Ser Thr Asn Val Leu
        35                  40                  45

Gly Glu Ala Lys Lys Leu Asn Glu Ser Gln Ala Pro Lys
```

50 55 60

<210> 3
<211> 58
<212> PRT
<213> Staphylococcus aureus

<400> 3

Ala Asp Asn Asn Phe Asn Lys Glu Gln Gln Asn Ala Phe Tyr Glu Ile
1 5 10 15

Leu Asn Met Pro Asn Leu Asn Glu Glu Gln Arg Asn Gly Phe Ile Gln
20 25 30

Ser Leu Lys Asp Asp Pro Ser Gln Ser Ala Asn Leu Leu Ala Glu Ala
35 40 45

Lys Lys Leu Asn Glu Ser Gln Ala Pro Lys
50 55

<210> 4
<211> 58
<212> PRT
<213> Staphylococcus aureus

<400> 4

Ala Asp Asn Lys Phe Asn Lys Glu Gln Gln Asn Ala Phe Tyr Glu Ile
1 5 10 15

Leu His Leu Pro Asn Leu Asn Glu Glu Gln Arg Asn Gly Phe Ile Gln
20 25 30

Ser Leu Lys Asp Asp Pro Ser Gln Ser Ala Asn Leu Leu Ala Glu Ala
35 40 45

Lys Lys Leu Asn Asp Ala Gln Ala Pro Lys
50 55

<210> 5
<211> 58
<212> PRT
<213> Staphylococcus aureus

<400> 5

Ala Asp Asn Lys Phe Asn Lys Glu Gln Gln Asn Ala Phe Tyr Glu Ile
1 5 10 15

Leu His Leu Pro Asn Leu Thr Glu Glu Gln Arg Asn Gly Phe Ile Gln
20 25 30

Ser Leu Lys Asp Asp Pro Ser Val Ser Lys Glu Ile Leu Ala Glu Ala
        35                  40                  45

Lys Lys Leu Asn Asp Ala Gln Ala Pro Lys
    50                  55

<210> 6
<211> 116
<212> PRT
<213> Artificial Sequence

<220>
<223> C domain mutant

<400> 6

Ala Asp Asn Arg Phe Asn Arg Glu Gln Gln Asn Ala Phe Tyr Glu Ile
1               5                   10                  15

Leu His Leu Pro Asn Leu Thr Glu Glu Gln Arg Asn Ala Phe Ile Gln
            20                  25                  30

Arg Leu Arg Asp Asp Pro Ser Val Ser Arg Glu Ile Leu Ala Glu Ala
        35                  40                  45

Gln Arg Leu Asn Asp Ala Gln Ala Pro Lys Ala Asp Asn Arg Phe Asn
    50                  55                  60

Arg Glu Gln Gln Asn Ala Phe Tyr Glu Ile Leu His Leu Pro Asn Leu
65                  70                  75                  80

Thr Glu Glu Gln Arg Asn Ala Phe Ile Gln Arg Leu Arg Asp Asp Pro
                85                  90                  95

Ser Val Ser Arg Glu Ile Leu Ala Glu Ala Gln Arg Leu Asn Asp Ala
            100                 105                 110

Gln Ala Pro Lys
        115

<210> 7
<211> 116
<212> PRT
<213> Artificial Sequence

<220>
<223> C domain mutant

<400> 7

Ala Asp Asn Gln Phe Asn Arg Glu Gln Gln Asn Ala Phe Tyr Glu Ile
1               5                   10                  15

```
Leu His Leu Pro Asn Leu Thr Glu Glu Gln Arg Asn Ala Phe Ile Gln
            20                  25                  30

Arg Leu Arg Asp Asp Pro Ser Val Ser Arg Glu Ile Leu Ala Glu Ala
        35                  40                  45

Gln Arg Leu Asn Asp Ala Gln Ala Pro Lys Ala Asp Asn Gln Phe Asn
    50                  55                  60

Arg Glu Gln Gln Asn Ala Phe Tyr Glu Ile Leu His Leu Pro Asn Leu
65                  70                  75                  80

Thr Glu Glu Gln Arg Asn Ala Phe Ile Gln Arg Leu Arg Asp Asp Pro
                85                  90                  95

Ser Val Ser Arg Glu Ile Leu Ala Glu Ala Gln Arg Leu Asn Asp Ala
            100                 105                 110

Gln Ala Pro Lys
        115
```

<210> 8
<211> 359
<212> DNA
<213> Artificial Sequence

<220>
<223> synthetic DNA

<400> 8

```
ctgcagataa ccgttttaac cgtgaacaac aaaacgcttt ctacgaaatc ctgcacttgc        60

caaaccttac tgaagaacaa cgtaatgctt tcatccaacg tctgcgcgat gatccatctg       120

tatcccgtga aattttggca gaggctcaac gacttaacga cgctcaggcg cctaaggcag       180

ataaccgttt taaccgtgaa caacaaaacg ctttctacga aatcctgcac ttgccaaacc       240

ttactgaaga acaacgtaat gctttcatcc aacgtctgcg cgatgatcca tctgtatccc       300

gtgaaatttt ggcagaggct caacgactta acgacgctca ggcgcctaag taatctaga        359
```

<210> 9
<211> 359
<212> DNA
<213> Artificial Sequence

<220>
<223> synthetic DNA

<400> 9

```
ctgcagataa ccaatttaac cgtgaacaac aaaacgcttt ctacgaaatc ctgcacttgc        60
```

```
caaaccttac tgaagaacaa cgtaatgctt tcatccaacg tctgcgcgat gatccatctg      120

tatcccgtga aattttggca gaggctcaac gacttaacga cgctcaggcg cctaaggcag      180

ataaccaatt taaccgtgaa caacaaaacg ctttctacga aatcctgcac ttgccaaacc      240

ttactgaaga acaacgtaat gctttcatcc aacgtctgcg cgatgatcca tctgtatccc      300

gtgaaatttt ggcagaggct caacgactta acgacgctca ggcgcctaag taatctaga       359


<210>  10
<211>  290
<212>  PRT
<213>  Artificial Sequence

<220>
<223>  C domain mutant

<400>  10

Ala Asp Asn Arg Phe Asn Arg Glu Gln Gln Asn Ala Phe Tyr Glu Ile
1               5                   10                  15


Leu His Leu Pro Asn Leu Thr Glu Glu Gln Arg Asn Ala Phe Ile Gln
            20                  25                  30


Arg Leu Arg Asp Asp Pro Ser Val Ser Arg Glu Ile Leu Ala Glu Ala
        35                  40                  45


Gln Arg Leu Asn Asp Ala Gln Ala Pro Lys Ala Asp Asn Arg Phe Asn
    50                  55                  60


Arg Glu Gln Gln Asn Ala Phe Tyr Glu Ile Leu His Leu Pro Asn Leu
65                  70                  75                  80


Thr Glu Glu Gln Arg Asn Ala Phe Ile Gln Arg Leu Arg Asp Asp Pro
                85                  90                  95


Ser Val Ser Arg Glu Ile Leu Ala Glu Ala Gln Arg Leu Asn Asp Ala
            100                 105                 110


Gln Ala Pro Lys Ala Asp Asn Arg Phe Asn Arg Glu Gln Gln Asn Ala
        115                 120                 125


Phe Tyr Glu Ile Leu His Leu Pro Asn Leu Thr Glu Glu Gln Arg Asn
    130                 135                 140


Ala Phe Ile Gln Arg Leu Arg Asp Asp Pro Ser Val Ser Arg Glu Ile
145                 150                 155                 160


Leu Ala Glu Ala Gln Arg Leu Asn Asp Ala Gln Ala Pro Lys Ala Asp
                165                 170                 175
```

Asn Arg Phe Asn Arg Glu Gln Gln Asn Ala Phe Tyr Glu Ile Leu His
180 185 190

Leu Pro Asn Leu Thr Glu Glu Gln Arg Asn Ala Phe Ile Gln Arg Leu
195 200 205

Arg Asp Asp Pro Ser Val Ser Arg Glu Ile Leu Ala Glu Ala Gln Arg
210 215 220

Leu Asn Asp Ala Gln Ala Pro Lys Ala Asp Asn Arg Phe Asn Arg Glu
225 230 235 240

Gln Gln Asn Ala Phe Tyr Glu Ile Leu His Leu Pro Asn Leu Thr Glu
245 250 255

Glu Gln Arg Asn Ala Phe Ile Gln Arg Leu Arg Asp Asp Pro Ser Val
260 265 270

Ser Arg Glu Ile Leu Ala Glu Ala Gln Arg Leu Asn Asp Ala Gln Ala
275 280 285

Pro Lys
290

<210> 11
<211> 881
<212> DNA
<213> Artificial Sequence

<220>
<223> synthetic DNA

<400> 11
ctgcagataa ccgttttaac cgtgaacaac aaaacgcttt ctacgaaatc ctgcacttgc 60

caaaccttac tgaagaacaa cgtaatgctt tcatccaacg tctgcgcgat gatccatctg 120

tatcccgtga aattttggca gaggctcaac gacttaacga cgctcaggcg cctaaggctg 180

ataaccgctt caaccgagaa caacaaaacg ctttttatga aatccttcac ctgccaaatc 240

ttacagaaga acaacgcaac gcattcattc aacgcttgcg tgatgaccct tccgttagcc 300

gagagatcct ggctgaagca caacgtttga atgatgcgca agcaccaaaa gctgataatc 360

gattcaaccg tgaacaacaa aatgcattct acgaaatctt gcaccttcct aacctgactg 420

aagagcagcg taacgctttt atccagcgtt tgagagacga tccatctgtc tcccgtgaaa 480

ttctcgcaga agcgcaacgc ctgaacgatg ctcaagctcc gaaagcagac aaccgtttca 540

atcgcgaaca gcaaaacgcg ttttatgaaa ttctgcatct tccaaacttg acagaggaac 600

aacgcaatgc tttcatccaa cgactgcgtg atgatccgag cgtttctcga gaaatcttgg 660

ctgaagcaca acgtctgaac gacgctcaag ctccaaaagc ggataacaga tttaaccgtg 720

aacaacaaaa tgctttctac gagatcttgc accttccgaa cctgactgaa gaacaacgta 780

acgcatttat tcagagattg cgcgatgacc catccgtaag ccgtgagatc ctggcagaag 840

ctcaacgttt gaatgatgca caagctccaa aataatctag a 881

<210> 12
<211> 30
<212> DNA
<213> Artificial Sequence

<220>
<223> primer 1

<400> 12
ttcgctgcag ataaccaatt taaccgtgaa 30

<210> 13
<211> 30
<212> DNA
<213> Artificial Sequence

<220>
<223> primer 2

<400> 13
actatctaga ttattttgga gcttgtgcat 30

<210> 14
<211> 116
<212> PRT
<213> Artificial Sequence

<220>
<223> C-G29A.2d

<400> 14

Ala Asp Asn Lys Phe Asn Lys Glu Gln Gln Asn Ala Phe Tyr Glu Ile
1 5 10 15

Leu His Leu Pro Asn Leu Thr Glu Glu Gln Arg Asn Ala Phe Ile Gln
20 25 30

Ser Leu Lys Asp Asp Pro Ser Val Ser Lys Glu Ile Leu Ala Glu Ala
35 40 45

Lys Lys Leu Asn Asp Ala Gln Ala Pro Lys Ala Asp Asn Lys Phe Asn
50 55 60

Lys Glu Gln Gln Asn Ala Phe Tyr Glu Ile Leu His Leu Pro Asn Leu
65 70 75 80

Thr Glu Glu Gln Arg Asn Ala Phe Ile Gln Ser Leu Lys Asp Asp Pro
85 90 95

Ser Val Ser Lys Glu Ile Leu Ala Glu Ala Lys Lys Leu Asn Asp Ala
100 105 110

Gln Ala Pro Lys
115

<210> 15
<211> 351
<212> DNA
<213> Artificial Sequence

<220>
<223> C-G29A.2d

<400> 15
gcagataaca aatttaacaa agaacaacaa aacgctttct acgaaatcct gcacttgcca 60

aaccttactg aagaacaacg taatgctttc atccaatccc tgaaagatga tccatctgta 120

tccaaagaaa ttttggcaga ggctaaaaaa cttaacgacg ctcaggcgcc taaggctgat 180

aacaaattca ataaagaaca gcaaaacgct tttatgaaa tccttcacct gccaaatctt 240

acagaagaac aacgcaacgc attcattcaa agcttgaagg atgacccttc cgttagcaaa 300

gagatcctgg ctgaagcaaa aaagttgaat gatgcgcaag caccaaaata a 351

<210> 16
<211> 115
<212> PRT
<213> Artificial Sequence

<220>
<223> C-G29A.2d-K07T

<400> 16

Ala Asp Asn Lys Phe Asn Thr Gln Gln Asn Ala Phe Tyr Glu Ile Leu
1 5 10 15

His Leu Pro Asn Leu Thr Glu Glu Gln Arg Asn Ala Phe Ile Gln Ser
20 25 30

Leu Lys Asp Asp Pro Ser Val Ser Lys Glu Ile Leu Ala Glu Ala Lys
35 40 45

Lys Leu Asn Asp Ala Gln Ala Pro Lys Ala Asp Asn Lys Phe Asn Lys
50 55 60

Glu Gln Gln Asn Ala Phe Tyr Glu Ile Leu His Leu Pro Asn Leu Thr

65 70 75 80

Glu Glu Gln Arg Asn Ala Phe Ile Gln Ser Leu Lys Asp Asp Pro Ser
85 90 95

Val Ser Lys Glu Ile Leu Ala Glu Ala Lys Lys Leu Asn Asp Ala Gln
100 105 110

Ala Pro Lys
115

<210> 17
<211> 351
<212> DNA
<213> Artificial Sequence

<220>
<223> C-G29A.2d-K07T

<400> 17
gcagataaca aatttaacac tgaacaacaa aacgctttct acgaaatcct gcacttgcca 60

aaccttactg aagaacaacg taatgctttc atccaatccc tgaaagatga tccatctgta 120

tccaaagaaa ttttggcaga ggctaaaaaa cttaacgacg ctcaggcgcc taaggctgat 180

aacaaattca ataaagaaca gcaaaacgct tttatgaaa tccttcacct gccaaatctt 240

acagaagaac aacgcaacgc attcattcaa agcttgaagg atgacccttc cgttagcaaa 300

gagatcctgg ctgaagcaaa aaagttgaat gatgcgcaag caccaaaata a 351

<210> 18
<211> 35
<212> DNA
<213> Artificial Sequence

<220>
<223> Primer 3

<400> 18
ttcgcatatg gcagataacc gttttaaccg tgaac 35

<210> 19
<211> 35
<212> DNA
<213> Artificial Sequence

<220>
<223> Primer 4

<400> 19
ttttctgcag ttattatttt ggagcttgtg catca 35

<210> 20
<211> 35

```
<212>  DNA
<213>  Artificial Sequence

<220>
<223>  Primer 5

<400>  20
ttcgcatatg gcagataacc aatttaaccg tgaac                              35


<210>  21
<211>  35
<212>  DNA
<213>  Artificial Sequence

<220>
<223>  Primer 6

<400>  21
ttcgcatatg gcagataacc gttttaacac tgaac                              35


<210>  22
<211>  35
<212>  DNA
<213>  Artificial Sequence

<220>
<223>  Primer 7

<400>  22
ttcgcatatg gcagataacc aatttaacac tgaac                              35
```

## Description

TECHNICAL FIELD

The present invention relates to a protein specifically binding to a target substance, a ligand affinity separation matrix on which the protein is immobilized, and a separation purification method using the matrix.

BACKGROUND ART

The antibody drugs developed so far are mainly monoclonal antibodies, which are massively produced by, for example, recombinant cell culture techniques. The "monoclonal antibodies" refer to antibodies that are produced by clones of a single antibody-producing cell. Monoclonal antibodies produced by cultured cells are purified by a variety of chromatographic techniques to prepare drugs. Among chromatographic processes, affinity chromatographic processes, particularly using Protein A affinity separation matrices, provide one-step, high-purity purification of antibodies from animal cell cultures, and thus are essential in the preparation of antibody drugs.

Protein A is a cell wall protein produced by the gram-positive bacterium *Staphylococcus aureus* and contains a signal sequence S, five immunoglobulin-binding domains (E domain, D domain, A domain, B domain, and C domain), and a cell wall-anchoring domain known as XM region (Non-Patent Literature 1).

Protein A affinity separation matrices are prepared by immobilizing Protein A onto separation matrices. Protein A affinity separation matrices are generally expensive products because, for one reason, Protein A itself is a recombinant protein that is produced by a cultured transformant containing a foreign gene and then highly purified. Techniques that allow us to produce Protein A with high culture productivity are industrially valuable and thus there is a need to develop them.

Many techniques have been developed for highly functionalizing Protein A by modifying it through protein engineering. Examples of Protein A engineering techniques include those for improving alkali resistance or antibody acid dissociation properties, and for improving antibody-binding capacity by mutagenesis into the immobilization site (Patent Literatures 1 to 4).

Generally, protein variants obtained by introducing a plurality of mutations to proteins can be expressed at lower levels in cells than the wild-type proteins. It is known that the reduction in protein expression level can be prevented by adding a highly expressed protein (tag sequence) to the N-terminus to produce the target protein as a fusion protein. Such a fusion protein, however, contains the unnecessary tag sequence, which may reduce the activity of the target protein. The potential loss of activity of the target protein can be reduced by removing the tag sequence from the fusion protein. This, however, requires an additional step of removing the tag sequence and is therefore inefficient as a method for producing a protein (Non-Patent Literature 2, Patent Literature 5).

CITATION LIST

PATENT LITERATURE

Patent Literature 1: WO 2003/080655
Patent Literature 2: EP 1123389
Patent Literature 3: WO 2011/118699
Patent Literature 4: WO 2012/133349
Patent Literature 5: WO 2009/101672

NON PATENT LITERATURE

Non-Patent Literature 1: Hober S. et al., "J. Chromatogr. B", 2007, 848, pp. 40-47
Non-Patent Literature 2: Smith, D. B. et al., "Gene", 1988, 67, pp. 31-40

## SUMMARY OF INVENTION

### TECHNICAL PROBLEM

An object of the present invention is to create an immunoglobulin-binding engineered protein that can be produced by a transformant with improved culture productivity.

### SOLUTION TO PROBLEM

In order to solve the above problem, the inventors have designed a numerous recombinant variants of Protein A, obtained the variants by protein engineering and genetic engineering techniques, and evaluated the culture productivity of the variants. As a result, the inventors have found that a protein containing two or more linked domains derived from Protein A in which a mutation is introduced near the N-terminus of the domain closest to the N-terminus can be produced by a transformant with improved culture productivity, without deteriorating the properties of Protein A such as antibody-binding capacity and alkali resistance. This finding has led to the completion of the present invention.

Specifically, the present invention relates to a protein, having two or more amino acid sequences derived from any of the E, D, A, B, and C domains of Protein A of SEQ ID NOs:1 to 5, wherein in the domain-derived amino acid sequence closest to the N-terminus, one or more amino acid residues corresponding to position 4 and/or position 7 of the C domain are substituted by amino acid residues other than Arg, and wherein the protein can be produced by a transformant with improved culture productivity as compared to before the substitution.

Preferably, in the domain-derived amino acid sequence closest to the N-terminus, an amino acid residue corresponding to position 4 of the C domain is substituted by Gln, Ala, Gly, Leu, Met, Phe, Pro, Val, Trp, Asn, Cys, Ser, Thr, Tyr, Asp, Glu, His, or Lys.

Preferably, in the domain-derived amino acid sequence closest to the N-terminus, an amino acid residue corresponding to position 7 of the C domain is substituted by Met, Phe, Asn, Gln, Thr, Tyr, His, or Lys.

Preferably, in the domain-derived amino acid sequence second closest to the N-terminus or subsequent ones, Lys is substituted by an amino acid residue other than Lys.

Preferably, at least 90% of the following amino acid residues are retained: Gln-9, Gln-10, Phe-13, Tyr-14, Leu-17, Pro-20, Asn-21, Leu-22, Gln-26, Arg-27, Phe-30, Ile-31, Leu-34, Pro-38, Ser-39, Leu-45, Leu-51, Asn-52, Gln-55, and Pro-57, wherein the residue numbers indicated are for the C domain.

The present invention further relates to a DNA, encoding the protein.

The present invention further relates to a vector, including the DNA.

The present invention further relates to a transformant, produced by transforming a host cell with the vector.

The present invention further relates to a method for producing the protein, the method including using the transformant, or a cell-free protein synthesis system including the DNA.

The present invention further relates to an affinity separation matrix, including the protein as an affinity ligand immobilized on a carrier made of a water-insoluble base material.

Preferably, the affinity separation matrix binds to a protein containing an immunoglobulin Fc region.

In a preferred embodiment of the affinity separation matrix, the protein containing an immunoglobulin Fc region is an immunoglobulin G or an immunoglobulin G derivative.

The present invention further relates to a method for preparing the affinity separation matrix, the method including immobilizing the protein as an affinity ligand onto a carrier made of a water-insoluble base material.

The present invention further relates to a method for purifying a protein containing an immunoglobulin Fc region, the method including adsorbing a polypeptide containing an immunoglobulin Fc region onto the affinity separation matrix.

### ADVANTAGEOUS EFFECTS OF INVENTION

The protein of the present invention can be produced by a transformant with improved culture productivity and therefore can be produced economically and efficiently.

## BRIEF DESCRIPTION OF DRAWINGS

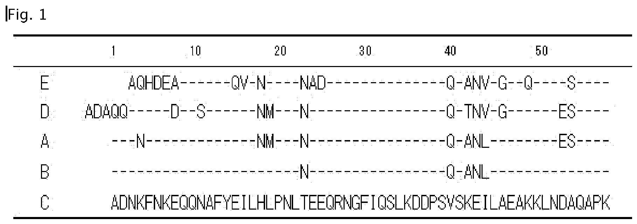

Fig. 1 shows a table for comparison of the sequences of the E, D, A, B, and C domains of Protein A from *Staphylococcus* sp.

## DESCRIPTION OF EMBODIMENTS

The protein of the present invention is a protein having two or more amino acid sequences derived from any of the E, D, A, B, and C domains of Protein A of SEQ ID NOs:1 to 5, wherein in the domain-derived amino acid sequence closest to the N-terminus, one or more amino acid residues corresponding to position 4 and/or position 7 of the C domain are substituted by amino acid residues other than Arg, and wherein the protein can be produced by the transformant with improved culture productivity as compared to before the substitution.

Substitutions of amino acid residues are denoted herein with the code for the wild-type or non-mutated type amino acid residue, followed by the position number of the substitution, followed by the code for changed amino acid residue. For example, a substitution of Ala for Gly at position 29 is represented by G29A.

The term "protein" is intended to include any molecule having a polypeptide structure and also encompass fragmented polypeptide chains and polypeptide chains linked by peptide bonds.

In general, the term "domain" refers to a higher- order protein structural unit having a sequence that consists of several tens to hundreds of amino acids, enough to fulfill a certain physicochemical or biochemical function. The domain as used herein particularly refers to a domain that binds to a protein containing an immunoglobulin Fc region.

The domain-derived amino acid sequence refers to an amino acid sequence before the amino acid substitution. The domain-derived amino acid sequence is not limited only to the wild-type amino acid sequences of the E, D, A, B, and C domains of Protein A, and may include any amino acid sequence partially engineered by amino acid substitution, insertion, deletion, or chemical modification, provided that it forms a protein having the ability to bind to an Fc region. Examples of the domain-derived amino acid sequence include the amino acid sequences of the E, D, A, B, and C domains of *Staphylococcus* Protein A of SEQ ID NOs: 1 to 5. Examples also include proteins having amino acid sequences obtained by introducing a substitution of Ala for Gly corresponding to position 29 of the C domain into the E, D, A, B, and C domains of Protein A. In addition, the Z domain produced by introducing A1V and G29A mutations into the B domain corresponds to the domain-derived amino acid sequence because it also has the ability to bind to an Fc region. The domain-derived amino acid sequence is preferably a domain having high chemical stability or a variant thereof. These domains can be aligned as shown in Fig. 1. For example, the residue corresponding position 31 of the C domain corresponds to that at the same position 31 of the A or B domain, at position 29 of the E domain, and at position 34 of the D domain.

The amino acid sequence derived from any of the domains is preferably an amino acid sequence obtained by introducing into any of the amino acid sequences of SEQ ID Nos:1 to 5 an amino acid substitution that meets at least one of the following conditions (1) to (4):

(1) the amino acid residue in the corresponding domain that corresponds to position 29 of the C domain is Ala, Val, Leu, Ile, Phe, Tyr, Trp, Thr, Ser, Asp, Glu, Arg, His, or Met;
(2) the amino acid residue in the corresponding domain that corresponds to position 33 of the C domain is Leu, Ile, Phe, Tyr, Trp, Thr, Asp, Glu, Asn, Gln, Arg, His, or Met;
(3) the amino acid residue in the corresponding domain that corresponds to position 36 of the C domain is Leu, Ile, Phe, Tyr, Trp, Glu, Arg, His, or Met; and
(4) the amino acid residue in the corresponding domain that corresponds to position 37 of the C domain is Leu, Ile, Phe, Tyr, Trp, Glu, Arg, His, or Met.

The amino acid sequence derived from any of the domains is more preferably an amino acid sequence obtained by introducing into any of the amino acid sequences of SEQ ID Nos:1 to 5 an amino acid substitution that meets at least one of the following conditions (1) to (4):

(1) the amino acid residue in the corresponding domain that corresponds to position 29 of the C domain is Ala, Glu, or Arg;
(2) the amino acid residue in the corresponding domain that corresponds to position 33 of the C domain is Leu, Thr, Glu, Gln, Arg, or His;
(3) the amino acid residue in the corresponding domain that corresponds to position 36 of the C domain is Ile or Arg; and
(4) the amino acid residue in the corresponding domain that corresponds to position 37 of the C domain is Leu, Ile, Glu, Arg, or His.

The domain-derived amino acid sequence preferably has at least 85%, more preferably at least 90%, still more preferably at least 95% sequence identity to any of the E, D, A, B, and C domains of Protein A of SEQ ID Nos:1 to 5.

The protein of the present invention has two or more amino acid sequences derived from any of the E, D, A, B, and C domains of Protein A of SEQ ID Nos:1 to 5. The number of domains in the protein of the present invention is two or more, preferably three or more, more preferably four or more, still more preferably five or more, even more preferably six or more. The number of domains in the protein of the present invention is 20 or less, preferably 10 or less, more preferably eight or less, still more preferably six or less.

In the present invention, the amino acid sequence of the domain closest to the N-terminus may be different from that of the domain(s) connected to the C-terminal end of the former domain. In the case where a plurality of domains are connected to the C-terminal end of the domain closest to the N-terminus, the domains other than the domain closest to the N-terminus may form a protein that includes a homopolymer (e.g. homodimer, homotrimer) consisting of linked single immunoglobulin-binding domains, terminated with Lys, or a protein that includes a heteropolymer (e.g. heterodimer, heterotrimer) consisting of linked different immunoglobulin-binding domains, terminated with Lys.

The monomeric proteins may be linked to each other by, for example, but not limited to: a method that does not use an amino acid residue as a linker; or a method that uses one or more amino acid residues. The number of amino acid residues used as the linker is not particularly limited, and is preferably such that the three-dimensional conformation of the monomeric proteins does not become unstable.

The term "linker" herein means a linker between domains, and refers to a linking portion of linked monomeric proteins (single domains), i.e., a region between the C-terminal region of a domain sequence closer to the N-terminus and the N-terminal region of a domain sequence closer to the C-terminus. In the case of a protein including N number of domains linked in tandem, the number of linkers in the protein is N-1. In other words, the "linker" herein refers to a region that consists of at least two amino acid residues, including the C-terminal amino acid of a domain closer to the N-terminus and the N-terminal amino acid of a domain closer to the C-terminus, which are connected to each other.

The linker may be an N-/C-terminal sequence of a domain that does not assume a specific secondary structure or that is located on the border between domains. In this case, the linker on the N-terminal side of an immunoglobulin G-binding domain of Protein A, for example, includes amino acid residues corresponding to positions 1 to 6, preferably positions 1 to 5, more preferably positions 1 to 4, still more preferably positions 1 to 3, even more preferably positions 1 to 2, of the C domain, and contains at least the N-terminal amino acid residue. It should be noted that although the E and D domains are different from the C domain in full length, their amino acid residues corresponding to the above-mentioned amino acids of the C domain correspond to the linker. Likewise, the linker on the C-terminal side of an immunoglobulin G-binding domain of Protein A, for example, includes amino acid residues corresponding to positions 55 to 58, preferably positions 56 to 58, more preferably positions 57 to 58, of the C domain, and contains at least the C-terminal amino acid residue at position 58.

The protein of the present invention having two or more domains is such that in the domain-derived amino acid sequence closest to the N-terminus, one or more amino acid residues corresponding to position 4 and/or position 7 of the C domain are substituted by amino acid residues other than Arg. The amino acid substitution means a mutation which deletes the original amino acid and adds a different type of amino acid to the same position.

In the domain-derived amino acid sequence closest to the N-terminus, the amino acid residue corresponding to position 4 of the C domain to be substituted is specifically an amino acid residue at position 2 of the E domain of Protein A of SEQ ID No:1, an amino acid residue at position 7 of the D domain of Protein A of SEQ ID No:2, an amino acid residue at position 4 of the A domain of Protein A of SEQ ID No:3, an amino acid residue at position 4 of the B domain of Protein A of SEQ ID No:4, or an amino acid residue at position 4 of the C domain of Protein A of SEQ ID No:5.

In the domain-derived amino acid sequence closest to the N-terminus, the amino acid residue corresponding to position 7 of the C domain to be substituted is specifically an amino acid residue at position 5 of the E domain of Protein A of SEQ ID No:1, an amino acid residue at position 10 of the D domain of Protein A of SEQ ID No:2, an amino acid residue at position 7 of the A domain of Protein A of SEQ ID No:3, an amino acid residue at position 7 of the B domain of Protein A of SEQ ID No:4, or an amino acid residue at position 7 of the C domain of Protein A of SEQ ID No:5. The term "corresponding" means that they are in the same column when the E, D, A, B, and C domains of Protein A are aligned as shown in Fig. 1.

In the domain-derived amino acid sequence closest to the N-terminus, the amino acid residue other than Arg after substitution of the amino acid residue corresponding to position 4 of the C domain may be Gln, Ala, Gly, Leu, Met, Phe, Pro, Val, Trp, Asn, Cys, Ser, Thr, Tyr, Asp, Glu, His, or Lys, preferably Gln, Ala, Gly, Leu, Met, Phe, Pro, Val, Trp, Asn, Cys, Ser, Thr, Tyr, Asp, Glu, His, or Lys, more preferably Gln or Lys.

In the domain-derived amino acid sequence closest to the N-terminus, the amino acid residue other than Arg after substitution of the amino acid residue corresponding to position 7 of the C domain may be Met, Phe, Asn, Gln, Thr, Tyr, His, or Lys, preferably Met, Phe, Asn, Gln, Thr, Tyr, His, or Lys, more preferably Thr or Lys.

In the domain-derived amino acid sequence closest to the N-terminus, the amino acid substitutions at positions corresponding to positions 4 and 7 of the C domain may be introduced alone or in combination. Preferred combinations of mutations are the following combinations (1) to (4):

(1) Position 4: Lys, position 7: Lys;
(2) Position 4: Gln, position 7: Lys;
(3) Position 4: Gln, position 7: Thr;
(4) Position 4: Lys, position 7: Thr.

In the domain-derived amino acid sequence closest to the N-terminus, the amino acid residues other than those corresponding to positions 4 and 7 of the C domain are not particularly limited as long as the protein can be produced by the transformant with improved culture productivity as compared to before substitution. Moreover, the domain-derived amino acid sequence second closest to the N-terminus and subsequent ones are not particularly limited either as long as the protein can be produced by the transformant with improved culture productivity as compared to before substitution.

The domain-derived amino acid sequence closest to the N-terminus, except for positions corresponding to positions 4 and 7 of the C domain, and the domain-derived amino acid sequence second closest to the N-terminus and subsequent ones may be functional variants that are free of Lys, except for linkers between domains and the C-terminus. Examples of such variants include C-K04R/K07R/G29A/S33R/K35R/K42R/K49Q/K50R.5d of SEQ ID No:8 in the Sequence Listing of WO 2012/133349. The Lys refers to six Lys residues in the E, D, A, or B domain of Protein A that correspond to positions 4, 7, 35, 49, 50, and 58 of the C domain. Only for the C domain of Protein A, it refers to seven Lys residues at positions 4, 7, 35, 42, 49, 50, and 58. However, it is not limited to the above numbers if the non-mutated amino acid sequences are sequences that have undergone deletion, such as sequences containing deletions at positions 1 to 4.

The amino acid residues in the domain-derived amino acid sequence closest to the N-terminus, except for positions corresponding to positions 4 and 7 of the C domain, and the domain-derived amino acid sequence second closest to the N-terminus and subsequent ones may be any amino acid residues such as wild-type amino acid residues, non-protein-forming amino acid residues, or non-natural amino acid residues. For production by genetic engineering, wild-type amino acid residues can be suitably used. However, amino acid residues having in a side chain a functional group that is highly reactive in a coupling reaction for immobilization, such as cysteine (Cys) having a thiol group (-SH) in a side chain, are unsuitable as amino acid residues used for substitution.

The domain-derived amino acid sequence closest to the N-terminus, except for positions corresponding to positions 4 and 7 of the C domain, and the domain-derived amino acid sequence second closest to the N-terminus and subsequent ones may contain amino acid substitutions that improve various functions. Examples of such amino acid substitutions include amino acid substitutions which substitute amino acids other than Ala for one or more Gly residues in an amino acid sequence derived from any of the E, D, A, B, and C domains of Protein A, as disclosed in WO 2010/110288. Examples also include amino acid substitutions which introduce at least one amino acid substitution into the amino acid residues at positions 31 to 37 of the A, B, or C domain, amino acid residues at positions 29 to 35 of the E domain, or amino acid residues at positions 34 to 40 of the D domain in an amino acid sequence derived from at least one domain selected from the E, D, A, B, and C domains of Protein A, as disclosed in WO 2011/118699. These amino acid substitutions can reduce affinity for Fab regions to improve antibody elution properties.

Shown below are specific examples of amino acid substitutions that may be contained in the domain-derived amino acid sequence closest to the N-terminus, except for positions corresponding to positions 4 and 7 of the C domain, and the domain-derived amino acid sequence second closest to the N-terminus and subsequent ones.

(1) A substitution of Arg, Gln, or Asn, preferably Arg, for Lys in the D, B, or C domain, except the domain closest to the N-terminus, that corresponds to position 4 of the C domain.

(2) A substitution of Asp, Arg, or Glu, preferably Arg, for Lys in the D, A, B, or C domain, except the domain closest to the N-terminus, that corresponds to position 7 of the C domain.

(3) A substitution of Arg, Ile, or His, preferably Arg or His, for Lys in the E, D, A, B, or C domain that corresponds to position 35 of the C domain.

(4) A substitution of Arg for Lys corresponding to position 42 in the C domain.

(5) A substitution of Arg or Gln for Lys in the D, A, B, or C domain that corresponds to position 49 of the C domain.

(6) A substitution of Arg or His, preferably Arg, for Lys in the E, D, A, B, or C domain that corresponds to position 50 of the C domain.

(7) A substitution of Arg or Gly, preferably Arg, for Lys in the E, D, A, B, or C domain that corresponds to position 58 of the C domain. However, if the amino acid residue in question is at (near) the C-terminus of the ligand, Lys may be left without introducing a substitution as described above.

In the domain-derived amino acid sequence second closest to the N-terminus or subsequent ones, Lys is preferably substituted by an amino acid residue other than Lys. The number of Lys residues substituted by amino acid residues other than Lys, among the Lys residues corresponding to positions 4, 7, 35, 42, 49, 50, and 58 of the C domain, is preferably one or more, more preferably two or more, still more preferably three or more, even more preferably four or more, particularly preferably five or more, most preferably six or more, and even most preferably all Lys residues are substituted.

The domain-derived amino acid sequence second closest to the N-terminus or subsequent ones in which Lys is substituted by an amino acid residue other than Lys may, for example, be the following amino acid sequence: RF$X_1X_2$EQQNAFYEILH$X_3$PNLTEEQRN$X_4$FIQ$X_5$L$X_6X_7X_8$PSVSRE$X_9$LAEA$X_{10}X_{11}$LND AQAP$X_{12}$ wherein $X_1$ = D, E,

N, or Q; $X_2$ = E or R; $X_3$ = L, M, or I; $X_4$ = A, E, F, R, Y, or W; $X_5$ = D, E, H, I, L, Q, R, S, T, or V; $X_6$ = H, I, or R; $X_7$ = D, I, or R; $X_8$ = D or E; $X_9$ = I, L, or V; $X_{10}$ = R or Q; $X_{11}$ = H or R; $X_{12}$ = R, G, or K, as disclosed in WO 2012/133342. Moreover, it may be an amino acid sequence derived from any of the E, D, A, B, and C domains of Protein A which contains amino acid substitutions for all lysine residues (Lys) and which is terminated with Lys, as disclosed in WO 2012/133349. Moreover, it may be an amino acid sequence including two or more amino acid sequences derived from any domain selected from the E, D, A, B, and C domains of Protein A which contain amino acid substitutions for all lysine residues (Lys), wherein the amino acid sequences are connected to each other through a linker, and at least one linker contains a lysine residue (Lys) or a cysteine residue (Cys), as disclosed in WO 2014/046278.

In the domain-derived amino acid sequence closest to the N-terminus, the domain-derived amino acid sequence second closest to the N-terminus or subsequent ones, at least half of the substitutions for Lys are preferably substitutions with arginine (Arg). This is because Arg is a basic amino acid having similar properties to Lys, and a substitution of Lys with Arg causes a relatively small effect on the properties of the whole protein.

In the protein of the present invention, preferably at least 90%, more preferably at least 95%, of the following 20 amino acid residues are retained: Gln-9, Gln-10, Phe-13, Tyr-14, Leu-17, Pro-20, Asn-21, Leu-22, Gln-26, Arg-27, Phe-30, Ile-31, Leu-34, Pro-38, Ser-39, Leu-45, Leu-51, Asn-52, Gln-55, and Pro-57 (the residue numbers indicated are for the C domain).

Moreover, the amino acid sequence of the whole protein preferably has at least 85%, more preferably at least 90%, still more preferably at least 95% sequence identity to the amino acid sequence before mutagenesis. The sequence identity of amino acid sequences can be analyzed by a person skilled in the art by direct comparison of the sequences; specifically, it may be analyzed using commercially available sequence analysis software, for example.

The protein of the present invention may be a fusion protein produced by fusion with another protein having a different function. Examples of the fusion protein include, but are not limited to, those fused with albumin or GST (glutathione S-transferase). The protein of the present invention may also be one fused with a nucleic acid (e.g. a DNA aptamer), a drug (e.g. an antibiotic), or a polymer (e.g. polyethylene glycol (PEG)).

The present invention also relates to a DNA having a base sequence encoding the protein obtained as above. The DNA may be any DNA having a base sequence that is translated into the amino acid sequence of the protein. Such a DNA can be obtained by common known techniques, such as polymerase chain reaction (hereinafter abbreviated as PCR). Alternatively, it can be synthesized by known chemical synthesis techniques or may be available from DNA libraries. A codon in the base sequence of the DNA may be replaced with a degenerate codon, and the base sequence is not necessarily the same as the original base sequence, provided that the coding base sequence is translated into the same amino acids.

Site-directed mutagenesis for modifying the base sequence of the DNA may be performed by, for example, recombinant DNA technology or PCR as follows.

Specifically, in the case of mutagenesis by recombinant DNA technology, for example, if there are suitable restriction enzyme recognition sequences on both sides of a mutagenesis target site in the gene encoding the protein of the present invention, a cassette mutagenesis method can be used in which these restriction enzyme recognition sequences are cleaved with the restriction enzymes to remove a region containing the mutagenesis target site, and a DNA fragment in which only the target site is mutated by chemical synthesis or other methods is then inserted.

In the case of site-directed mutagenesis by PCR, for example, a double primer method can be used in which PCR is performed using a double-stranded plasmid encoding the protein as a template and two synthetic oligo primers containing complementary mutations in the + and - strands.

The DNA encoding the protein having two or more amino acid sequences derived from any of the E, D, A, B, and C domains of Protein A of SEQ ID Nos:1 to 5 can be obtained by ligating DNAs encoding a single domain. For example, the ligation of DNAs may be accomplished by introducing an appropriate restriction enzyme recognition sequence into a base sequence, fragmenting the sequence with the restriction enzyme, and ligating the double-stranded DNA fragments using a DNA ligase. A single restriction enzyme recognition sequence or a plurality of different restriction enzyme recognition sequences may be introduced.

The method for preparing the DNA encoding the protein having two or more amino acid sequences derived from any of the E, D, A, B, and C domains of Protein A of SEQ ID Nos:1 to 5 is not limited to the methods described above. For example, it may be prepared by applying any of the mutagenesis methods to a DNA encoding Protein A (e.g., see WO 2006/004067). Here, if the base sequences each encoding a monomeric protein in the DNA encoding a polymeric protein are the same, then homologous recombination of the DNA may be induced in transformed host cells. For this reason, the ligated DNAs encoding a monomeric protein preferably have 90% or lower, more preferably 85% or lower base sequence identity.

The "vector" of the present invention includes a base sequence encoding the above-described protein or a partial amino acid sequence thereof, and a promoter that is operably linked to the base sequence to function in a host cell. Typically, the vector can be constructed by linking or inserting the above-described gene encoding the protein into an appropriate vector. The vector used for insertion of the gene is not particularly limited, provided that it is capable of autonomous replication in a host cell. The vector may be a plasmid DNA or phage DNA. For example, when *Escherichia coli* is used as a host cell, examples of the vector include pQE vectors (QIAGEN), pET vectors (Merck), and pGEX vectors (GE Healthcare, Japan). Examples of plasmid vectors useful for the expression of *Brevibacillus* genes include the known *Bacillus subtilis* vector pUB110 and pHY500 (JP H02-31682 A), pNY700 (JP H04-278091 A), pNU211R2L5 (JP H07-170984 A), pHT210 (JP H06-133782 A), and the shuttle vector pNCMO2 between *Escherichia coli* and *Brevibacillus* (JP 2002-238569 A).

The protein of the present invention can be obtained as a fusion protein with a known protein that serves to assist protein expression or facilitate purification. Examples of such proteins include, but are not limited to, maltose-binding protein (MBP) and glutathione S-transferase (GST). The fusion protein can be produced using a vector that contains the DNA of the present invention and a DNA encoding a protein such as MBP or GST ligated to each other.

The "transformant" of the present invention can be produced by introducing the recombinant vector of the present invention into a host cell. The recombinant DNA may be introduced into a host cell by, for example, but not limited to: a calcium ion method, an electroporation method, a spheroplast method, a lithium acetate method, an agrobacterium infection method, a particle gun method, or a polyethylene glycol method. Moreover, the obtained gene function may be expressed in a host cell, for example, by incorporating the gene obtained in the present invention into a genome (chromosome).

The host cell is not particularly limited, and examples suitable for low-cost mass production are *Escherichia coli, Bacillus subtilis,* and bacteria (eubacteria) of genera including *Brevibacillus, Staphylococcus, Streptococcus, Streptomyces,* and *Corynebacterium.*

The protein of the present invention may be produced by culturing the above-described transformant in a medium to produce and accumulate the protein of the present invention in the cultured cells (including the periplasmic space thereof) or in the culture medium (extracellularly), and collecting the desired protein from the culture.

Alternatively, the protein of the present invention may be produced by culturing the above-described transformant in a medium to produce and accumulate a fusion protein containing the protein of the present invention in the cultured cells (including the periplasmic space thereof) or in the culture medium (extracellularly), collecting the fusion protein from the culture, cleaving the fusion protein with an appropriate protease, and collecting the desired protein.

The transformant of the present invention can be cultured in a medium according to common methods for culturing host cells. The medium used to culture the transformant may be any medium that allows for high yield and high efficiency production of the protein. Specifically, carbon and nitrogen sources such as glucose, sucrose, glycerol, polypeptone, meat extracts, yeast extracts, and casamino acids may be used. In addition, the medium may optionally be supplemented with inorganic salts such as potassium salts, sodium salts, phosphates, magnesium salts, manganese salts, zinc salts, or iron salts. When an auxotrophic host cell is used, nutritional substances necessary for its growth may be added. Moreover, antibiotics such as penicillin, erythromycin, chloramphenicol, or neomycin may optionally be added.

Furthermore, a variety of known protease inhibitors, i.e. phenylmethane sulfonyl fluoride (PMSF), benzamidine, 4-(2-aminoethyl)-benzenesulfonyl fluoride (AEBSF), antipain, chymostatin, leupeptin, pepstatin A, phosphoramidon, aprotinin, and ethylenediaminetetraacetic acid (EDTA), and/or other commercially available protease inhibitors may be added at appropriate concentrations in order to reduce the degradation or molecular-size reduction of the target protein caused by host-derived proteases present inside or outside the cells.

Furthermore, in order to ensure accurate folding of the protein of the present invention, molecular chaperones such as GroEL/ES, Hsp70/DnaK, Hsp90, or Hsp104/ClpB may be used (for example, they may be allowed to coexist with the protein of the present invention by, for example, co-expression or incorporation into a fusion protein). Other methods for ensuring accurate folding of the protein of the present invention may also be used such as, but not limited to, adding an additive for assisting accurate folding to the medium or culturing at low temperatures.

Examples of media that can be used to culture the transformant obtained using *Escherichia coli* as a host include LB medium (1% triptone, 0.5% yeast extract, 1% NaCl), 2xYT medium (1.6% triptone, 1.0% yeast extract, 0.5% NaCl), and D medium (1.6% Select soytone, 1.0% yeast extract, 0.5% NaCl).

Examples of media that can be used to culture the transformant obtained using *Brevibacillus* as a host include TM medium (1% peptone, 0.5% meat extract, 0.2% yeast extract, 1% glucose, pH 7.0), 2SL medium (4% peptone, 0.5% yeast extract, 2% glucose, pH 7.2), and A medium (3.0% polypeptone, 0.5% yeast extract, 3% glucose, 0.01% magnesium sulfate, 0.001% iron sulfate, 0.001% manganese chloride, 0.0001% zinc chloride).

The cell is aerobically cultured at a temperature of 15°C to 42°C, preferably 20°C to 37°C, for several hours to several days under aeration and stirring conditions to accumulate the protein of the present invention in the cultured cells (including the periplasmic space thereof) or in the culture medium (extracellularly), followed by recovery of the protein. In some cases, the cell may be cultured anaerobically without air.

In the case where the recombinant protein is secreted, the produced recombinant protein can be recovered after the culture by separating the cultured cells from the supernatant containing the secreted protein by a common separation method such as centrifugation or filtration.

Also, in the case where the protein is accumulated in the cultured cells (including the periplasmic space thereof), the protein produced and accumulated in the cells can be recovered, for example, by collecting the cells from the culture medium, e.g. via centrifugation or filtration, followed by disrupting the cells, e.g. via sonication or French press, and/or solubilizing the protein with, for example, a surfactant.

The protein of the present invention can be purified by methods such as affinity chromatography, cation or anion exchange chromatography, and gel filtration chromatography, used alone or in an appropriate combination.

Whether the purified product is the target protein may be confirmed by common methods such as SDS polyacrylamide gel electrophoresis, N-terminal amino acid sequencing, or Western blot analysis.

The protein of the present invention can also be produced using a cell-free protein synthesis system including the DNA. Examples of such cell-free protein synthesis systems include synthesis systems derived from procaryotic cells, plant cells, or higher animal cells.

The protein of the present invention is characterized in that it can be produced by the transformant with improved culture productivity as compared to before the amino acid substitution. The term "culture productivity" can be rephrased as "protein expression level" and refers to the amount per unit volume or per cell of the target recombinant protein produced by culturing the transformant as described above. For example, for secretory production, it may be the concentration of the target protein in the culture supernatant, while in the case where the target recombinant protein is produced in the cells, it can be expressed as the weight of the target protein per cell count or per cell weight. The expression "the protein can be produced by the transformant with improved culture productivity as compared to before the amino acid substitution" specifically means that when a transformant producing a non-mutated protein and a transformant producing the protein containing amino acid substitution are cultured under the same conditions, and then evaluated by the later-described "Method for evaluating culture productivity", the relative value is higher by at least 5%, preferably by at least 10%, more preferably by at least 20%.

The culture productivity can be evaluated as follows, for example.

[Method for evaluating culture productivity]

A transformant producing a non-mutated protein (control) and a transformant producing the protein containing amino acid substitution (evaluation sample) are prepared using the same host and the same transformation method, are cultured under the same conditions. For example, they are aerobically cultured in a medium suited for the host selected from the media mentioned above at an appropriate culture temperature (e.g. 20°C to 37°C) under aeration and stirring conditions for several hours to several days to accumulate the protein of the present invention in the cultured cells (including the periplasmic space thereof) or in the culture medium (extracellular secretion), followed by recovery of the protein. The amount of the protein in the cultured cells or culture medium is quantitated. The quantitation may be carried out by high performance liquid chromatography (HPLC), for example. In HPLC analysis, an appropriate pretreatment is performed on the cultured cells or culture medium. For example, in the case where the protein is accumulated in the cells, the cells are disrupted with a device such as a sonicator and then centrifuged to remove the cell residues, followed by filtration. In the case where the protein is extracellularly secreted, the cells are separated from the culture medium by centrifugation, followed by filtration. The control, the evaluation sample, and a reference (a highly purified protein having a predetermined concentration) are analyzed by HPLC, and the concentration of the reference and the analytical data (chromatographic areas) are used to calculate the concentration of the protein in the control or evaluation sample, which is then used to calculate the relative value using the following (Equation 1). The culture productivity is considered improved if the relative value of the evaluation sample is higher by at least 5%, preferably by at least 10%, more preferably by at least 20% than that of the control.

(Equation 1) Relative value (%) = [protein concentration of control]/[protein concentration of evaluation sample]

The protein of the present invention can be used as an affinity ligand having affinity for an immunoglobulin. An affinity separation matrix can be prepared by a method including immobilizing the protein of the present invention as an affinity ligand onto a carrier made of a water-insoluble base material.

The term "affinity ligand" means a substance (functional group) that selectively captures (binds to) a target molecule from a mixture of molecules by virtue of a specific affinity between the molecules such as antigen-antibody binding, and refers herein to a protein that specifically binds to an immunoglobulin. The term "ligand" as used alone herein is synonymous with "affinity ligand".

The "immunoglobulin binding properties" can be tested using, for example, but not limited to, biosensors such as Biacore system (GE Healthcare, Japan) based on the surface plasmon resonance principle. The measurement may be carried out under any conditions that allow detection of a binding signal corresponding to the binding of Protein A to an immunoglobulin Fc region. The properties can be easily evaluated at a (constant) temperature of 20°C to 40°C and a neutral pH of 6 to 8.

Examples of binding parameters that can be used include affinity constant (KA) and dissociation constant (KD) (Nagata et al., "Real-time analysis of biomolecular interactions", Springer-Verlag Tokyo, 1998, page 41). The affinity constant of the protein of the present invention for Fc may be determined in an experimental system using Biacore system in which human IgG is immobilized on a sensor chip, and each domain variant is added to a flow channel at a temperature of 25°C and a pH of 7.4. The protein according to the present invention may suitably be a protein having an affinity constant (KA) for human IgG of at least $1\times10^5$ ($M^{-1}$), more preferably at least $1\times10^6$ ($M^{-1}$), still more preferably $1\times10^7$ ($M^{-1}$).

In order to provide an affinity ligand that has high chemical stability under alkali conditions, the amino acid sequence before mutagenesis is preferably an amino acid sequence derived from the C domain of SEQ ID No:5. Moreover, the amino acid sequence before mutagenesis is more preferably an amino acid sequence derived from the C domain of SEQ ID No:5 in which the amino acid residue corresponding to position 29 is any amino acid residue selected from Ala, Arg, Glu, Ile, Leu, Met, Phe, Trp, and Tyr. Furthermore, at least half of the amino acid residues introduced to all Lys residues are preferably substitutions with Arg, and more preferably all of them are substitutions with Arg. In the case where all of them are substitutions with Arg, the chemical stability under alkali conditions can be improved as compared to before the mutagenesis.

The chemical stability under alkali conditions may be determined using binding activity to an immunoglobulin or material stability of a polypeptide itself as an index. In the case where material stability of a polypeptide itself is used as an index, the chemical stability under alkali conditions can be evaluated, for example, by electrophoresis in which electrophoresis bands of the polypeptide before and after an alkali treatment are compared. Specifically, comparison of chemical stability may be made by performing standard SDS-PAGE to analyze band intensity via densitometry. If chemical stability is indicated based on the band intensities analyzed via densitometry, the polypeptide of the present invention, after being left in a 0.5 M sodium hydroxide aqueous solution at 25°C for 24 hours, preferably has a band intensity of 50% or higher, more preferably 60% or higher, still more preferably 70% or higher, most preferably 80% or higher, of that before the treatment.

Examples of the carrier made of a water-insoluble base material used in the present invention include inorganic carriers such as glass beads and silica gel; organic carriers such as synthetic polymers (e.g. cross-linked polyvinyl alcohol, cross-linked polyacrylate, cross-linked polyacrylamide, cross-linked polystyrene) and polysaccharides (e.g. crystalline cellulose, cross-linked cellulose, cross-linked agarose, cross-linked dextran); and composite carriers formed by combining these carriers, such as organic-organic or organic-inorganic composite carriers. Examples of commercially available products include GCL2000 (porous cellulose gel), Sephacryl S-1000 (prepared by covalently cross-linking allyl dextran with methylene bisacrylamide), Toyopearl (methacrylate carrier), Sepharose CL4B (cross-linked agarose carrier), and Cellufine (cross-linked cellulose carrier), although the water-insoluble carrier used in the present invention is not limited to the carriers listed above.

In view of the purpose and method of using the affinity separation matrix, the water-insoluble carrier used in the present invention should have a large surface area and is preferably a porous material having a large number of fine pores of an appropriate size. The carrier may be in any form such as bead, monolith, fiber, film (including hollow fiber) or other optional forms.

The ligand may be immobilized by any method that allows the ligand to be covalently bonded to the carrier via the $\varepsilon$-amino group of lysine on the ligand by a conventional coupling process. Moreover, even if some ligands turn out to be immobilized on the carrier via the $\alpha$-amino group at the N-terminus, the effect of the present invention will not deteriorate because they are immobilized at the protein terminus. The coupling process may be carried out by reacting the carrier with cyanogen bromide, epichlorohydrin, diglycidyl ether, tosyl chloride, tresyl chloride, hydrazine, sodium periodate, or the like to activate the carrier (or introduce a reactive functional group into the carrier surface), and performing a coupling reaction between the carrier and the compound to be immobilized as a ligand to immobilize the ligand; or by adding a condensation reagent such as carbodiimide or a reagent having a plurality of functional groups in the molecule such as glutaraldehyde to a system containing the carrier and the compound to be immobilized as a ligand, followed by condensation and cross-linking for immobilization. Moreover, a spacer molecule consisting of a plurality of atoms may be introduced between the ligand and the carrier, or alternatively, the ligand may be directly immobilized onto the carrier.

The affinity separation matrix of the present invention can be used to separate and purify a protein containing an immunoglobulin Fc region by affinity column chromatography purification techniques. As described earlier, the regions to which immunoglobulin-binding domains bind are broadly specified as Fab regions (particularly Fv regions) and Fc regions. However, since the conformation of antibodies is already known, it is possible to further alter (e.g. fragmentize) the Fab or Fc regions while maintaining the conformation of the regions to which Protein A binds by protein engineering techniques. Accordingly, the present invention is not limited to immunoglobulin molecules containing Fab and Fc regions sufficiently, and derivatives thereof. Therefore, the term "protein containing an immunoglobulin Fc region" refers to a protein containing an Fc region portion to which Protein A binds, and it does not have to contain the complete Fc region as long as Protein A is able to bind to the protein.

Typical examples of the protein containing an immunoglobulin Fc region include, but are not limited to, immunoglobulin G and immunoglobulin G derivatives. The term "immunoglobulin G derivative" is a generic term for engineered artificial proteins to which Protein A can bind, such as chimeric immunoglobulin G in which the domains of human immunoglobulin G are partially replaced and fused with immunoglobulin G domains of another biological species, humanized immunoglobulin G in which complementarity determining regions (CDRs) of human immunoglobulin G are replaced and fused with antibody CDRs of another biological species, immunoglobulin G whose Fc region has a molecularly altered sugar chain, and artificial immunoglobulin G in which the Fv and Fc regions of human immunoglobulin G are fused.

These proteins containing an immunoglobulin Fc region can be purified according to affinity column chromatographic purification techniques using existing commercial Protein A columns. Specifically, a buffer containing the protein containing an immunoglobulin Fc region is adjusted to be neutral, and the resulting solution is passed through an affinity column filled with the affinity separation matrix of the present invention to adsorb the protein containing an immunoglobulin Fc region. Next, an appropriate volume of pure buffer is passed through the affinity column to wash the inside of the column. At this point, the desired protein containing an immunoglobulin Fc region remains adsorbed on the affinity separation matrix of the present invention in the column. Subsequently, an acidic buffer (which may contain a substance for promoting dissociation from the matrix) adjusted to an appropriate pH is passed through the column to elute the desired protein containing an immunoglobulin Fc region, whereby high-level purification can be achieved.

The affinity separation matrix of the present invention can be reused by passing therethrough a pure buffer having an appropriate strong acidity or strong alkalinity which does not completely impair the functions of the ligand compound and the carrier base material (or optionally a solution containing an appropriate modifying agent or an organic solvent) for washing.

The present invention further relates to a protein obtained by a separation method using the affinity separation matrix. The protein is preferably a protein containing an immunoglobulin Fc region. The protein obtained by using the affinity separation matrix is obtained as a high-purity, high-concentration solution, and has properties that maintain its inherent activity such as ability to bind to an antigen, without impairing it.

## EXAMPLES

The following description is offered to illustrate the present invention in greater detail by reference to examples, but the scope of the present invention is not limited to these examples. In the examples, operations such as recombinant DNA production and engineering were performed in accordance with the following textbooks, unless otherwise noted: (1) T. Maniatis, E. F. Fritsch, J. Sambrook, "Molecular Cloning/A Laboratory Manual", 2nd edition (1989), Cold Spring Harbor Laboratory (USA); (2) Masami Muramatsu, "Lab Manual for Genetic Engineering", 3rd edition (1996), Maruzen Co., Ltd. The materials such as reagents and restriction enzymes used in the examples were commercially available products, unless otherwise specified.

Proteins obtained in the examples are represented by "an alphabetical letter identifying the domain - an introduced mutation (wild for the wild type)". For example, the wild-type C domain of Protein A is represented by "C-wild", and a C domain variant containing G29E mutation is represented by "C-G29E". Variants containing two mutations together are represented by indicating both with a slash. For example, a C domain variant containing G29E and S13L mutations is represented by "C-G29E/S13L". Proteins consisting of a plurality of single domains linked together are represented by adding a period (.) followed by the number of linked domains followed by "d". For example, a protein consisting of five linked C domain variants containing G29E and S13L mutations is represented by "C-G29E/S13L.5d". In the case of proteins containing a mutation in the domain closest to the N-terminus, and a different mutation in the domain second closest to the N-terminus and subsequent domains, the mutations in the second and subsequent domains are represented as described above, and the mutation in the domain closest the N-terminus is added after the number of linked domains. For example, a protein consisting of five linked C domain variants and containing K04Q mutation in the domain closest to the N-terminus and K04R mutation in the domains second to fifth closest to the N-terminus is represented by "C-K04R.5d-K04Q".

### (Example 1) Evaluation of C domain variant containing amino acid substitution at position 4

Expression plasmids of C domain variants of Protein A obtained by substituting Lys residues other than the C-terminal Lys-58 by other amino acids (C-K04R/K07R/G29A/S33R/K35R/K42R/K49Q/K50R.2d (SEQ ID No:6) and C-K04Q/K07R/G29A/S33R/K35R/K42R/K49Q/K50R.2d (SEQ ID No:7)) were prepared by the following procedure.

The total synthesis of a DNA (SEQ ID No:8) encoding C-K04R/K07R/G29A/S33R/K35R/K42R/K49Q/K50R.2d in which PstI and XbaI recognition sites were added to the 5' and 3' ends, respectively, was outsourced to Eurofins Genomics K.K. It was subcloned into an expression plasmid, which was then digested with the restriction enzymes PstI and XbaI (Takara Bio, Inc.), and the obtained DNA was ligated to a *Brevibacillus* expression vector pNCMO2 (Takara Bio, Inc.) digested with the same restriction enzymes to construct an expression plasmid in which a DNA encoding the amino acid sequence of SEQ ID No:6 was inserted into the *Brevibacillus* expression vector pNCMO2. The plasmid was prepared using *Escherichia coli* JM109.

The total synthesis of a DNA (SEQ ID No:9) encoding C-K04Q/K07R/G29A/S33R/K35R/K42R/K49Q/K50R.2d (SEQ ID No:7) was similarly performed to prepare a plasmid.

*Brevibacillus choshinensis* SP3 (Takara Bio, Inc.) was transformed with the obtained plasmids, and the recombinant strains secreting the C domain variants were grown. Each recombinant strain was cultured with shaking at 30°C for three days in 30 mL of A medium (3.0% polypeptone, 0.5% yeast extract, 3% glucose, 0.01% magnesium sulfate, 0.001% iron sulfate, 0.001% manganese chloride, 0.0001% zinc chloride) containing 60 μg/mL neomycin. After the culture, the culture medium was sampled to analyze turbidity at 600 nm using a spectrophotometer. Moreover, the cells were removed from the culture medium by centrifugation (15,000 rpm, 25°C, five minutes) to measure the concentration of the C domain variant in the culture supernatant by high performance liquid chromatography. The culture results are shown in Table 1.

The variant C-K04Q/K07R/G29A/S33R/K35R/K42R/K49Q/K50R.2d was produced with higher culture productivity (higher concentration of the C domain variant in the culture supernatant) than C-K04R/K07R/G29A/S33R/K35R/K42R/K49Q/K50R.2d. The amino acid substitution at position 4 of the C domain resulted in improved culture productivity of the transformant.

[Table 1]

| C domain variant | Turbidity | Concentration of C domain variant (g/L) |
|---|---|---|
| C-K04R/K07R/G29A/S33R/K35R/K42R/K49Q/K50R.2d | 32 | 1.1 |
| C-K04Q/K07R/G29A/S33R/K35R/K42R/K49Q/K50R.2d | 33 | 1.7 |

(Example 2) Evaluation of C domain variant containing amino acid substitution at position 4 of N-terminal domain

The total synthesis of a DNA (SEQ ID No:11) encoding C-K04R/K07R/G29A/S33R/K35R/K42R/K49Q/K50R.5d (SEQ ID No:10) in which PstI and XbaI recognition sites were added to the 5' and 3' ends, respectively, was outsourced to Eurogentec. The DNA was used to prepare a plasmid and grow a recombinant strain as in Example 1.

PCR was performed using the DNA encoding C-K04R/K07R/G29A/S33R/K35R/K42R/K49Q/K50R.5d as a template and Primer 1: 5'-TTCGCTGCAGATAACCAATTTAACCGTGAA-3' (SEQ ID No:12) and Primer 2: 5'-ACTATCTAGATTATTTTGGAGCTTGTGCAT-3' (SEQ ID No:13) to amplify a DNA fragment encoding C-K04R/K07R/G29A/S33R/K35R/K42R/K49Q/K50R.5d-K04Q in which K04Q substitution was introduced only into the N-terminal domain of C-K04R/K07R/G29A/S33R/K35R/K42R/K49Q/K50R.5d. The DNA fragment was digested with restriction enzymes PstI and XbaI, and ligated to a *Brevibacillus* expression vector pNCMO2 digested with the same restriction enzymes to construct an expression plasmid in which the DNA encoding C-K04R/K07R/G29A/S33R/K35R/K42R/K49Q/K50R.5d-K04Q was inserted into the *Brevibacillus* expression vector pNCMO2. The plasmid was sequenced using BigDye Terminator Cycle Sequencing Kit (Life Technologies Japan Ltd.) and Applied Biosystems 3130xl genetic analyzer (Life Technologies Japan Ltd.) to confirm that the mutation was introduced only at the target position. Similarly to Example 1, *Brevibacillus choshinensis* SP3 was transformed and the recombinant strain secreting C-K04R/K07R/G29A/S33R/K35R/K42R/K49Q/K50R.5d-K04Q was grown.

The transformant producing C-K04R/K07R/G29A/S33R/K35R/K42R/K49Q/K50R.5d and the transformant producing C-K04R/K07R/G29A/S33R/K35R/K42R/K49Q/K50R.5d-K04Q were cultured as in Example 1 and the culture media were evaluated (n = 3). The results (averages with n = 3) are shown in Table 2.

The variant C-K04R/K07R/G29A/S33R/K35R/K42R/K49Q/K50R.5d-K04Q was produced with higher culture productivity than C-K04R/K07R/G29A/S33R/K35R/K42R/K49Q/K50R.5d. The amino acid substitution only at position 4 of the N-terminal domain, among the five domains, resulted in improved culture productivity.

[Table 2]

| C domain variant | Turbidity | Concentration of C domain variant (g/L) |
|---|---|---|
| C-K04R/K07R/G29A/S33R/K35R/K42R/K49Q/K50R.5d | 42 | 2.0 |
| C-K04R/K07R/G29A/S33R/K35R/K42R/K49Q/K50R.5d-K04Q | 36 | 2.6 |

(Example 3) Evaluation of culture of C domain variant containing amino acid substitutions at position 4/position 7 of N-terminal domain (codon engineering)

Similarly to Example 1, four mutants were produced which had different arginine codons at position 4 or 7 of the N-terminal domain in C-K04R/K07R/G29A/S33R/K35R/K42R/K49Q/K50R.5d. Also, mutants were produced which had different glutamine codons at position 4 of the N-terminal side domain in C-K04R/K07R/G29A/S33R/K35R/K42R/K49Q/K50R.5d-K04Q. For PCR primers, the total synthesis of five DNAs (Table 3) obtained by substituting a base sequence encoding the amino acid at position 4 or 7 in the sequence of Primer 1 shown in Example 1 was outsourced to Eurofins Genomics K.K. Transformants producing the variants were cultured as in Example 1 and the culture media were evaluated (n = 3).
The results (averages with n = 3) are shown in Table 3.

There was not much difference in culture productivity of C-K04R/K07R/G29A/S33R/K35R/K42R/K49Q/K50R.5d before and after the codon engineering, and the culture productivity of C-K04R/K07R/G29A/S33R/K35R/K42R/K49Q/K50R.5d-K04Q was still high even when the codon was engineered. These results show that culture productivity is not affected by codon engineering, but by amino acid substitution.

[Table 3]

| Mutation in N-terminal domain | | Turbidity | Concentration of C domain variant (g/L) |
|---|---|---|---|
| Position 4 (codon) | Position 7 (codon) | | |
| R (CGT) | R (CGT) | 40 | 1.9 |
| R (CGC) | R (CGT) | 42 | 1.9 |
| R (AGA) | R (CGT) | 43 | 2.0 |
| R (CGT) | R (CGC) | 41 | 2.0 |
| R (CGT) | R (AGA) | 41 | 1.9 |
| Q (CAA) | R (CGT) | 39 | 2.6 |
| Q (CAC) | R (CGT) | 38 | 2.5 |

(Example 4) Evaluation of culture of C domain variant containing amino acid substitution at position 4/position 7 of N-terminal domain

Similarly to Example 1, 37 variants were produced which contained amino acid substitutions at position 4 or 7 of the N-terminal domain in C-K04R/K07R/G29A/S33R/K35R/K42R/K49Q/K50R.5d (Table 4). For PCR primers, the total synthesis of 37 DNAs obtained by substituting a base sequence encoding the amino acid at position 4 or 7 in the sequence of Primer 1 shown in Example 1 was outsourced to Eurofins Genomics K.K. The variants were cultured and evaluated as in Example 1.

The culture productivity of the variants was evaluated based on the values relative to the concentration of the C domain variant in the culture supernatant of C-K04R/K07R/G29A/S33R/K35R/K42R/K49Q/K50R.5d taken as 100%. The results are shown in Table 4.

The culture productivity was improved for the multiple variants, as well as C-K04R/K07R/G29A/S33R/K35R/K42R/K49Q/K50R.5d-K04Q shown in Example 1.

[Table 4]

| No. | Mutation in N-terminal domain | | Culture productivity relative value (%) |
|---|---|---|---|
| | Position 4 | Position 7 | |
| Before mutagenesis | R | R | 100 |
| 1 | A | R | 111 |
| 2 | G | R | 114 |
| 3 | I | R | 102 |
| 4 | L | R | 118 |
| 5 | M | R | 133 |
| 6 | F | R | 112 |
| 7 | P | R | 116 |

(continued)

| No. | Mutation in N-terminal domain | | Culture productivity relative value (%) |
|---|---|---|---|
| | Position 4 | Position 7 | |
| 8 | V | R | 156 |
| 9 | W | R | 186 |
| 10 | N | R | 180 |
| 11 | C | R | 219 |
| 12 | S | R | 117 |
| 13 | T | R | 149 |
| 14 | Y | R | 132 |
| 15 | D | R | 196 |
| 16 | E | R | 258 |
| 17 | H | R | 110 |
| 18 | K | R | 143 |
| 19 | R | A | 86 |
| 20 | R | G | 81 |
| 21 | R | I | 86 |
| 22 | R | L | 75 |
| 23 | R | M | 224 |
| 24 | R | F | 128 |
| 25 | R | P | 86 |
| 26 | R | V | 60 |
| 27 | R | W | 98 |
| 28 | R | N | 169 |
| 29 | R | C | 82 |
| 30 | R | Q | 137 |
| 31 | R | S | 98 |
| 32 | R | T | 217 |
| 33 | R | Y | 106 |
| 34 | R | D | 84 |
| 35 | R | E | 100 |
| 36 | R | H | 136 |
| 37 | R | K | 205 |

(Example 5) Evaluation of mini jar culturing of C domain variant containing amino acid substitution at position 4/position 7 of N-terminal domain

Transformants producing the N-terminal variants C-K04R/K07R/G29A/S33R/K35R/K42R/K49Q/K50R.5d-K04Q (hereinafter, referred to as K04Q) and C-K04R/K07R/G29A/S33R/K35R/K42R/K49Q/K50R.5d-K07T (hereinafter, referred to as K07T), which were prepared in Examples 2 and 3 and exhibited improved culture productivity, and C-K04R/K07R/G29A/S33R/K35R/K42R/K49Q/K50R.5d (hereinafter, referred to as control) were aerobically cultured using a 5 L mini jar in C medium (1.5% peptone, 0.8% yeast extract, 2.0% glucose, 0.38% phosphate, 0.04% magnesium sulfate heptahydrate, 0.004% manganese sulfate pentahydrate, 0.004% iron sulfate heptahydrate, 0.0004% zinc sulfate heptahydrate, 750 ppm Disfoam CC-118, pH 7.2; glucose continuously added (up to 3.8%) from 6 h after the start of the culture). The culture temperature was 34°C from the start of the culture to 13.5 h and then shifted to 30°C. The pH of the medium was controlled in the range of pH 7.0 to pH 7.2 from the start to the end of the culture. The pH for the control was controlled in the range of pH 7.9 to pH 8.0 to provide higher culture productivity. The culture media were sampled over time to measure the turbidity and the concentration of the C domain variant in the culture supernatant as in Example 1. The results after 48 hours of culture are shown in Table 5.

The N-terminal variants were also produced with higher culture productivity in mini jar culture than the non-mutated protein.

[Table 5]

| C domain variant | Turbidity | Concentration of C domain variant (g/L) |
|---|---|---|
| Control | 81 | 4.0 |
| K04Q | 57 | 5.5 |
| K07T | 58 | 5.9 |

(Example 6) Evaluation of culture of C domain variant containing multiple amino acid substitutions at position 4/position 7 of N-terminal domain

Similarly to Example 3, four variants were prepared which contained multiple amino acid substitutions at positions 4 and 7 of the N-terminal domain in C-K04R/K07R/G29A/S33R/K35R/K42R/K49Q/K50R.5d (Table 6). For PCR primers, the total synthesis of four DNAs obtained by substituting base sequences encoding the amino acids at positions 4 and 7 in the sequence of Primer 1 shown in Example 1 was outsourced to Eurofins Genomics K.K. The variants were cultured and evaluated as in Example 1.

The culture productivity for each variant was evaluated based on the C domain variant concentration in the culture supernatant of C-K04R/K07R/G29A/S33R/K35R/K42R/K49Q/K50R.5d (n = 3). The results (averages with n = 3) are shown in Table 6.

Each multiple substitution variant was produced with improved culture productivity as compared to C-K04R/K07R/G29A/S33R/K35R/K42R/K49Q/K50R.5d.

[Table 6]

| C domain variant | Turbidity | Concentration of C domain variant (g/L) |
|---|---|---|
| C-KG4R/K07R/G29NS33R/K36R/K42R/K49Q/K50R.5d | 42 | 2.0 |
| C-K04R/K07R/G29/VS33R/K35R/K42R/K49Q/K50R.5d-4K7K | 38 | 2.3 |
| C-K04R/K07R/G29A/S33R/K35R/K42R/K49Q/K50R.5d-4Q7K | 37 | 2.5 |
| C-K04R/K07R/G29A/S33R/K35R/K42R/K49Q/K50R.5d-4Q7T | 34 | 2.9 |
| C-K04R/KO7R/G29NS33R/K35R/K42R/K49Q/K50R.5d-4K7T | 35 | 2.9 |

(Example 7) Evaluation of culture of C domain variant containing amino acid substitution at position 7 of N-terminal domain

Plasmids were constructed using a DNA (SEQ ID No:15) encoding the C domain variant C-G29A.2d (SEQ ID No:14) and a DNA (SEQ ID No:17) encoding the N-terminal domain variant C-G29A.2d-K07T (SEQ ID No:16) and transformants were grown as in Example 1. The total synthesis of each DNA in which PstI and XbaI recognition sites were added to the 5' and 3' ends, respectively, was outsourced to Eurofins Genomics K.K. Each transformant was cultured and evaluated as in Example 1 (n = 3). The results (averages with n = 3) are shown in Table 7.

The variant C-G29A.2d-K07T was produced with higher culture productivity than C-G29A.2d. This shows that the culture productivity-improving effect of N-terminal mutation is not limited to C domain variants of C-K04R/K07R/G29A/S33R/K35R/K42R/K49Q/K50R.

[Table 7]

| C domain variant | Turbidity | Concentration of C domain variant (g/L) |
|---|---|---|
| C-G29A.2d | 37 | 2.5 |
| C-G29A2d-K07T | 35 | 2.7 |

(Example 8) Evaluation of culture in *Escherichia coli* of C domain variant containing amino acid substitution at position 4/position 7 of N-terminal domain

The total synthesis of a DNA (SEQ ID No:11) encoding C-K04R/K07R/G29A/S33R/K35R/K42R/K49Q/K50R.5d (SEQ ID No:10) in which PstI and XbaI recognition sites were added to the 5' and 3' ends, respectively, was outsourced to Eurogentec. PCR was performed using the DNA as a template, Primer 3: 5'-TTCGCATATGGCAGATAACCGTTTTAACCGTGAAC-3' (SEQ ID No:18), and Primer 4: 5'-TTTTCTGCAGTTATTATTTTGGAGCTTGTGCATCA-3' (SEQ ID No:19) to amplify the DNA in which Met and an NdeI recognition site were added to the 5' end and a PstI recognition site to the 3' end. Further, Primer 3 was replaced by Primer 5: 5'-TTCGCATATGGCAGATAACCAATTTAACCGTGAAC-3' (SEQ ID No:20), Primer 6: 5'-TTCGCATATGGCAGATAACCGTTTTAACACTGAAC-3' (SEQ ID No:21), and Primer 7: 5'-TTCGCATATGGCAGATAACCAATTTAACACTGAAC-3' (SEQ ID No:22) to amplify, by PCR, C-K04R/K07R/G29A/S33R/K35R/K42R/K49Q/K50R.5d-K04Q, C-K04R/K07R/G29A/S33R/K35R/K42R/K49Q/K50R.5d-K07T, and C-K04R/K07R/G29A/S33R/K35R/K42R/K49Q/K50R.5d-K04Q/K07T, respectively.

The obtained DNA fragments were digested with restriction enzymes NdeI and PstI, and ligated to an *Escherichia coli* expression vector pUCNT (WO 94/03613) digested with the same restriction enzymes to construct expression plasmids in which a DNA encoding each C domain variant was inserted into the *Escherichia coli* expression vector pUCNT. The base sequences in the plasmids were determined using BigDye Terminator Cycle Sequencing Kit (Life Technologies Japan Ltd.) and Applied Biosystems 3130xl genetic analyzer (Life Technologies Japan Ltd.) to confirm that the mutation was introduced only at the target position. *Escherichia coli* HB101 (Takara Bio, Inc.) was transformed with the obtained plasmids, and the transformants producing each C domain variant were grown.

The transformants producing each C domain variant were cultured with shaking at 37°C for 24 hours in 50 mL of D medium (1.6% Select soytone, 1.0% yeast extract, 0.5% NaCl) containing 100 μg/mL ampicillin. After the culture, the culture media were sampled to analyze turbidity at 600 nm using a spectrophotometer. Each culture medium was centrifuged to remove the supernatant and obtain the cells, which were then disrupted by an ultrasonic homogenizer, and the homogenate was centrifuged to obtain a cell-free extract. The concentration of the C domain variant in the cell-free extract was measured by high performance liquid chromatography. The culture was performed with n = 3. The results (averages with n = 3) are shown in Table 8.

Each N-terminal variant was also produced by a recombinant *Escherichia coli* with higher culture productivity than C-K04R/K07R/G29A/S33R/K35R/K42R/K49Q/K50R.5d. This shows that the culture productivity-improving effect of N-terminal mutation is not limited to the cases using *Brevibacillus* host cells.

[Table 8]

| C domain variant | Turbidity | Concentration of C domain variant (g/L) |
|---|---|---|
| C-K04R/K07R/G29A/S33R/K35R/K42R/K49Q/K60R.5d (control) | 18 | 0.8 |
| C-K04R/K07R/G29A/S33R/K35R/K42R/K49Q/K50R.5d-K04Q | 18 | 1.0 |
| C-K04R/K07R/G29A/S33R/K35R/K42R/K49Q/K50R.5d-K07Q | 20 | 0.9 |
| C-K04R/K07R/G29A/S33R/K35R/K42R/K49Q/K50R.5d-K04Q/K07T | 17 | 1.0 |

**Claims**

1. A protein, comprising two or more amino acid sequences derived from any of the E, D, A, B, and C domains of Protein A of SEQ ID NOs:1 to 5,
wherein in the domain-derived amino acid sequence closest to the N-terminus, one or more amino acid residues corresponding to position 4 and/or position 7 of the C domain are substituted by amino acid residues other than Arg, and
wherein the protein can be produced by a transformant with improved culture productivity as compared to before the substitution.

2. The protein according to claim 1,
wherein in the domain-derived amino acid sequence closest to the N-terminus, an amino acid residue corresponding to position 4 of the C domain is substituted by Gln, Ala, Gly, Leu, Met, Phe, Pro, Val, Trp, Asn, Cys, Ser, Thr, Tyr, Asp, Glu, His, or Lys.

3. The protein according to claim 1,
wherein in the domain-derived amino acid sequence closest to the N-terminus, an amino acid residue corresponding to position 7 of the C domain is substituted by Met, Phe, Asn, Gln, Thr, Tyr, His, or Lys.

4. The protein according to any one of claims 1 to 3,
wherein in the domain-derived amino acid sequence second closest to the N-terminus or subsequent ones, Lys is substituted by an amino acid residue other than Lys.

5. The protein according to any one of claims 1 to 4,
wherein at least 90% of the following amino acid residues are retained: Gln-9, Gln-10, Phe-13, Tyr-14, Leu-17, Pro-20, Asn-21, Leu-22, Gln-26, Arg-27, Phe-30, Ile-31, Leu-34, Pro-38, Ser-39, Leu-45, Leu-51, Asn-52, Gln-55, and Pro-57, wherein the residue numbers indicated are for the C domain.

**6.** A DNA, encoding the protein according to any one of claims 1 to 5.

**7.** A vector, comprising the DNA according to claim 6.

**8.** A transformant, produced by transforming a host cell with the vector according to claim 7.

**9.** A method for producing the protein according to any one of claims 1 to 5, the method comprising using the transformant according to claim 8, or a cell-free protein synthesis system comprising the DNA according to claim 6.

**10.** An affinity separation matrix, comprising
the protein according to any one of claims 1 to 5 as an affinity ligand immobilized on a carrier made of a water-insoluble base material.

**11.** The affinity separation matrix according to claim 10,
wherein the affinity separation matrix binds to a protein containing an immunoglobulin Fc region.

**12.** The affinity separation matrix according to claim 11,
wherein the protein containing an immunoglobulin Fc region is an immunoglobulin G or an immunoglobulin G derivative.

**13.** A method for preparing the affinity separation matrix according to any one of claims 10 to 12, the method comprising immobilizing the protein according to any one of claims 1 to 5 as an affinity ligand onto a carrier made of a water-insoluble base material.

**14.** A method for purifying a protein containing an immunoglobulin Fc region, the method comprising
adsorbing a polypeptide containing an immunoglobulin Fc region onto the affinity separation matrix according to any one of claims 10 to 12.

Fig. 1

| | 1 10 20 30 40 50 |
|---|---|
| E | AQHDEA------QV-N----NAD--------------Q-ANV-G--Q----S---- |
| D | ADAQQ-----D--S------NM---N----------------Q-TNV-G------ES---- |
| A | ---N-------------NM---N----------------Q-ANL--------ES---- |
| B | ----------------------N----------------Q-ANL-------------- |
| C | ADNKFNKEQQNAFYEILHLPNLTEEQRNGFIQSLKDDPSVSKEILAEAKKLNDAQAPK |

## INTERNATIONAL SEARCH REPORT

International application No.
PCT/JP2016/072649

A. CLASSIFICATION OF SUBJECT MATTER
*C12N15/09*(2006.01)i, *C07K1/22*(2006.01)i, *C07K14/31*(2006.01)i, *C12N1/15*(2006.01)i, *C12N1/19*(2006.01)i, *C12N1/21*(2006.01)i, *C12N5/10*(2006.01)i, *C12P21/02*(2006.01)i

According to International Patent Classification (IPC) or to both national classification and IPC

B. FIELDS SEARCHED

Minimum documentation searched (classification system followed by classification symbols)
C12N15/09, C07K1/22, C07K14/31, C12N1/15, C12N1/19, C12N1/21, C12N5/10, C12P21/02

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)
JSTPlus/JMEDPlus/JST7580(JDreamIII), CAplus/MEDLINE/EMBASE/BIOSIS(STN)

C. DOCUMENTS CONSIDERED TO BE RELEVANT

| Category* | Citation of document, with indication, where appropriate, of the relevant passages | Relevant to claim No. |
|---|---|---|
| A | JP 2010-504754 A (GE Healthcare Bio-Sciences AB.), 18 February 2010 (18.02.2010), entire text; particularly, claims; examples & US 2010/0048876 A1 claims; examples & WO 2008/039141 A1 & EP 2066419 A | 1-14 |
| A | JP 2006-304633 A (Apro Life Science Institute, Inc.), 09 November 2006 (09.11.2006), entire text; particularly, claims; examples (Family: none) | 1-14 |

☒ Further documents are listed in the continuation of Box C.　　☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 18 August 2016 (18.08.16) | 04 October 2016 (04.10.16) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No.
PCT/JP2016/072649

C (Continuation). DOCUMENTS CONSIDERED TO BE RELEVANT

| Category* | Citation of document, with indication, where appropriate, of the relevant passages | Relevant to claim No. |
|---|---|---|
| A | WO 2014/046278 A1 (Kaneka Corp.), 27 March 2014 (27.03.2014), entire text; particularly, claims; examples & US 2016/0168209 A claims; examples & EP 2899271 A1 | 1-14 |
| A | WO 2015/005859 A1 (GE Healthcare Bio-Sciences AB.), 15 January 2015 (15.01.2015), claims; examples & US 2016/0159855 A & EP 3019520 A & CN 105377880 A | 1-14 |
| A | JP 2015-77152 A (Kaneka Corp.), 23 April 2015 (23.04.2015), entire text; particularly, claims; examples & WO 2010/001960 A1 | 1-14 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2003080655 A
- EP 1123389 A
- WO 2011118699 A
- WO 2012133349 A
- WO 2009101672 A
- WO 2010110288 A
- WO 2012133342 A
- WO 2014046278 A
- WO 2006004067 A
- JP H0231682 A
- JP H07170984 A
- JP H06133782 A
- JP 2002238569 A
- WO 9403613 A


### Non-patent literature cited in the description


- **HOBER S. et al.** *J. Chromatogr. B,* 2007, vol. 848, 40-47
- **SMITH, D. B. et al.** *Gene,* 1988, vol. 67, 31-40
- **NAGATA et al.** Real-time analysis of biomolecular interactions. Springer-Verlag, 1998, 41
- **T. MANIATIS ; E. F. FRITSCH ; J. SAMBROOK.** Molecular Cloning/A Laboratory Manual. Cold Spring Harbor Laboratory, 1989
- **MASAMI MURAMATSU.** Lab Manual for Genetic Engineering. Maruzen Co., Ltd, 1996